# EUROPÄISCHE PATENTANMELDUNG

(11) **EP 1 911 513 A2**
(43) Veröffentlichungstag der Anmeldung: **16.04.2008**
(21) Anmeldenummer: 07019003.8
(22) Anmeldetag: 27.09.2007
(51) Int. Cl.: B01J 23/50, B01J 35/04, A61L 9/015, B01D 53/86, B01J 23/72, B01J 23/755, B01J 23/889, C02F 1/78, F24F 3/12

(54) **Katalytischer Ozon-Konverter**

(30) Priorität: 02.10.2006 DE 102006047011
(71) Anmelder: Girlich, Dieter, Dr., 01309 Dresden (DE); Lehmann, Jörg, Dipl.-Ing., 01723 Kesselsdorf (DE); Lerche, Christian, 01277 Dresden (DE)
(72) Erfinder: Girlich, Dieter, Dr., 01309 Dresden (DE); Lehmann, Jörg, Dipl.-Ing., 01723 Kesselsdorf (DE); Lerche, Christian, 01277 Dresden (DE)
(74) Vertreter: Kaufmann, Sigfrid

(57) **Zusammenfassung**

Die Erfindung betrifft einen Katalysator zur Zersetzung von Ozon in Dünsten, Rauchgasen und Dämpfen sowie in wässrigen Medien. Erfindungsgemäß wird für den Ozon-Katalysator als Katalysatorträger eine offenporige Schaumstruktur aus Metall, deren Stege aufgedickt wurden, oder eine Gitternetzstruktur aus Kunststoff, bestehend aus einem retikulierten, ihre Geometrie bestimmenden Schaumstoff, dessen Stege allseitig und gleichmäßig mit Kunststoffpulver beschichtet sind, verwendet. Vorteilhafte Verwendungen des Ozon-Katalysators sind in Trinkwasserkreisläufen, in technischen Wasseraufbereitungsanlagen, in Tischgeräten zum Geruchsabbau und Entkeimen von Luft in Wohnräumen, in Radiatoren, in Raumteilern, in Absaugungen für Küchendünste, Rauchgase und Dämpfe, in Druckern, Scannern und Kopierern sowie als Perkolstor in Duschköpfen oder Wasserventilen und in Filteranlagen in Schwimmbädern gegeben.

## Beschreibung

Die Erfindung betrifft einen Katalysator zur Zersetzung von Ozon in der Luft und in wässrigen Medien.

Ozon ist ein Molekül, das aus drei Sauerstoffatomen besteht. Es bildet sich auf natürlichem Wege, indem UV- und Höhenstrahlung auf Sauerstoffatome trifft. In technischen Anlagen wird Ozon durch UV-Lampen erzeugt und zum Bleichen von Papier, zum Entkeimen von Schwimmbädern, zum Reinigen von technischen Abwässern und Sterilisieren von Lüftungsanlagen und zum Abbau von Geruchs- und Schadstoffen in der Raumluft verwendet.

Ozon ist sehr reaktiv und daher äußerst giftig. Der Ozongehalt in der Abluft technischer Anlagen sollte deshalb den Wert von 50 µg/m³ nicht überschreiten und unterhalb der Geruchsschwelle liegen. Somit steht bei technischen Anwendungen die Aufgabe, dass überschüssiges Ozon in der Abluft durch Katalysatoren auf ein unter dieser Schwelle liegendes Maß abzubauen. Das Absorbieren von Ozon und Schadstoffen, z. B. durch Aktivkohle, ist durch die kurze Lebensdauer der Absorptionsmittel und der hohen Entsorgungskosten wirtschaftlich nicht zu betreiben. Deshalb haben sich seit einiger Zeit besonders Katalysatoren aus Metall oder Metalloxid, aber auch Katalysatoren mit einer Trägerstruktur aus Kunststoff, namentlich Polyurethan, durchgesetzt.

Ein katalytischer Konverter zum Entfernen von Ozon mit anodisierter Schicht und Überzug ist aus EP 1 112 117 B1 bekannt. Zudem sind Katalysatoren und Methoden für die Zersetzung von Ozon in EP 0 487 506 A1 vorgeschlagen; auch aus EP 0 501 003 B1 und WO 96/04988 können seit längerem Anregungen für den Aufbau und den Einsatz derartig bestimmter Katalysatoren entnommen werden.

Aus folgenden Schriften ist bekannt, Metallschäume bzw. metallbeschichtete PU-Schäume als Katalysatoren zur Zersetzung von Ozon bzw. von Kohlenwasserstoffen zu verwenden.

In DE 39 204 28 A1 wird ein Trägerkatalysator zur Zersetzung von Ozon in Gasen und insbesondere zur Reinigung von Atemluft beschrieben. Der Träger des Katalysators besteht hierbei aus einem geschäumten, offenporigen organischen Polymer, wie z. B. Polyurethan. Der Träger wird mit einer katalytisch aktiven Beschichtung aus Metallen, Übergangsmetallen oder deren Oxiden bzw. mit entsprechenden Gemischen versehen. Die Beschichtung kann z. B. durch ein Kontaktieren des Trägers mit einer Aufschlämmung der feingemahlenen, katalytischen Komponenten und ein anschließendes Trocken erfolgen.

Aus DE 29 38 046 A1 ist ein Katalysator zur Zersetzung von Ozon, der aus einem Kupfersubstrat, das mit einer katalytisch aktiven Schicht versehen ist, zu entnehmen. Der Träger kann aus geschäumtem oder teilchenförmigem Kupfer bzw. aus Kupferdrahtmaschen oder Kupferfilz bestehen. Alternativ dazu können auch teilchenförmiges Zirkonoxid, Zirkonoxidfasern, faserförmiger Zirkonoxidfilz oder Zirkonoxidgespinst verwendet werden. Die katalytische Schicht besteht aus einer Mischung aus Silber und Manganoxid.

Des Weiteren wird in DE 38 237 32 C2 ein Katalysator zum Kracken von Ozon offenbart, der aus einem Träger aus Keramikfasern, in den Manganoxid als katalytisch aktives Material eingelagert ist, besteht. Hierbei soll das Manganoxid Mikroporen mit einem Durchmesser von 100 - 2000 Å und einem Volumen von 0,2 cm³/g und der Träger freie Poren mit einem Durchmesser von 5000 Å und einem Volumen von 0,1 cm³/g aufweisen. Das Manganoxid wird durch Eintauchen des Trägers in eine wässrige Mangannitrat-Lösung, eine optionale Behandlung des Trägers mit Ammoniakgas, Trocknen in erhitzter Luft und anschließendes Kalzinieren bei 200 bis 400 °C in den Träger eingebracht.

In DE 199 27 562 A1 wird ein Vollmetallkatalysator für die Oxidation von kohlenmonoxid- kohlenwasserstoff- und rußhaltigen Gemischen in der Gasphase offenbart. Zur Herstellung des Katalysators wird eine, mindestens drei der Elemente Mangan, Nickel, Kupfer, Kobalt, Chrom, Molybdän, Wolfram, Titan, Zirkonium, Vanadium, Niob, Aluminium und Eisen enthaltende Legierung einer ersten oxidierenden, thermischen Behandlung unterzogen, die Oberfläche bzw. die oberflächennahen Bereich dotiert und die Legierung anschließend einer zweiten oxidieren, thermischen Behandlung (5 Minuten bis 3 Stunden bei einer Temperatur von 250 bis 1250°C) unterzogen.

Schließlich wird in EP 0 403 984 B1 ein Polymerschaum als Träger für einen Katalysator vorgeschlagen. Offenporiger Polyurethanschaum und dessen Derivate sind als Katalysatorträger für Temperaturen unter 50 °C wegen der offenporigen Struktur sehr gut geeignet. Gänzlich ungeeignet ist Polyurethan als Katalysatorträger allerdings für Fluide infolge seiner geringen Steifigkeit; hier ist Polyurethan nur in kleinen Abmessungen einsetzbar, größere Flächen können nur aufwändig durch eine Vielzahl kleiner Segmente abgedeckt werden.

Aufgabe der Erfindung ist es, einen Katalysator vorzuschlagen, der anforderungsgerecht Ozon abbaut und zugleich sich durch eine ausreichende Stabilität auszeichnet.

Diese Aufgabe wird dadurch gelöst, dass als Katalysatorträger eine offenporige Schaumstruktur aus Metall oder eine stabile Gitternetzstruktur aus Kunststoff verwendet wird, die jeweils mit katalytisch aktivem Material beschichtet ist.

Der Metallschaum besteht üblicherweise aus gegossenem Zinn, Zink, Aluminium oder Kupfer bzw. deren Legierungen. Bevorzugt ist ein Metallschaum verwendet, dessen Stege zielgerichtet aufgedickt sind, sodass er eine sehr stabile Struktur aufweist. Für besonders raue Einsatzgebiete ist es vorteilhaft, den Katalysator direkt aus dem katalytisch aktiven Material zu gießen. Hierfür kommen bevorzugt Silber, aber auch Legierungen mit Nickel-, Kupfer- und Manganbestandteilen zum Einsatz. Diese Materialien sind ansonsten auf den Metallschaum bzw. auf die stabile Gitternetzstruktur aus Kunststoff aufgedampft.

Die Kunststoffstruktur weist einen retikulierten, ihre Geometrie bestimmenden Schaumstoff, dessen Stege allseitig und gleichmäßig mit Kunststoffpulver beschichtet sind, auf, wobei der Schaumstoff bevorzugt ein so genannter harter Polyurethanschaumstoff ist, und zeichnet sich dadurch aus, dass die Kunststoffpulverschicht aus mehreren Einzelschichten aus gleichem und/oder unterschiedlichem Material besteht. Das Kunststoffbeschichtungspulver enthält bevorzugt Graphit und/oder Metall und/oder Keramik. Auf der Kunststoffpulverschicht können eine oder mehrere Schichten aus Metall und/oder Glas aufgebracht sein. Auf diese stabile Gitternetzstruktur aus Kunststoff sind die katalytisch aktiven Materialien aufgedampft.

Der erfindungsgemäße Katalysator kann z. B. in einen Trinkwasserkreislauf derart eingesetzt sein, dass ein offenporiger, gegossener, mit Silber bedampfter Metallschaum in Zylinderform, dessen Querschnitt der wasserdurchströmten Rohrleitung entspricht, in diese eingebracht und hierin in geeigneter Weise, z. B. mittels Schweißheftpunkte, dauerhaft oder lösbar, z. B. mittels Klemmelemente, befestigt wird.

Dem Wesen folgend kann auch ein Vollmetallkatalysator in der Reinigungsstufe einer technischen Wasseraufbereitung, wie sie in industriellen Fertigungen zur Gewährleistung gleich bleibender Wasserqualität üblich ist, eingebaut sein. In solch geschlossenen Wasserkreisläufen setzen sich Bakterien und Algen ab, die die Wasserqualität stark beeinträchtigen. Zur Unterdrückung von Algenwachstum wird das Wasser mit UV-Lampen bestrahlt und infolgedessen große Mengen von Ozon erzeugt. Zum Abbau überschüssigen Ozons wird der Vollmetallkatalysator aus einer NiCuMn-Legierung, der eine Porosität von 85 % und eine Porengröße von 4 mm aufweist, in den Kreislauf auf einer Länge von 150 bis 200 mm eingebaut.

Weiterhin ist erfindungsgemäß der Einsatz von versteiftem Polyurethan, das mit Silber beschichtet ist, in einem Tischgerät zum Geruchsabbau und Entkeimen der Luft in Wohnräumen vorgesehen. Durch eine Entladung über eine Nadelelektrode oder eine UV-Lampe wird Ozon erzeugt, organische Bestandteile der Luft oxidiert und gefiltert. Der Katalysator kann durch eine angelegte Spannung als elektrostatischer Filter benutzt werden. Auf Grund eines Wechselrahmens ist der Filter durch einfaches Spülen leicht zu reinigen.

Des Weiteren kann versteifter PU-Schaum oder Aluminiumschaum mit katalytisch aktiver Schicht als Hohlzylinder, beispielsweise mit einem Außendurchmesser von 350 mm, einem Innendurchmesser von 250 mm und einer Höhe von 200 mm, als Radiator betrieben werden. Die am Außendurchmesser weggeschleuderten Luftmoleküle erzeugen einen Unterdruck, der die Luft durch den Innendurchmesser nachströmen lässt. Die Struktur wirkt also als Ventilator und Katalysator in einem.

Zudem kann der versteifte Polyurethanschaum als Flächenelement in einem Raumteiler derart Anwendung finden, dass einerseits der Boden des Raumteilers Raumluft ansaugt und Ozon zumischt und andererseits der Luftaustritt über die Außenplatten des Raumteilers, die katalytisch aktiv beschichtet sind, erfolgt.

Weitere vorteilhafte Verwendungen des erfindungsgemäßen Katalysators sind:
- Absaugungen für Rauchgase, wie sie z. B. beim Schweißen entstehen, in Werkstätten
- Luftabsaugungen in Küchen mit Reinigung und Geruchsentfernung
- Verwendung als Perkolstor in einen Duschkopf oder Wasserventil
- Verwendung im Schwimmbad
- Verwendung in Druckern, Scannern, Kopierern
- Verwendung für den Abbau von Lösungsmitteln in Druckereien.

## Patentansprüche

1. Ozon-Katalysator, ausgeführt als ein Katalysatorträger, der mit katalytisch aktiven Materialien beschichtet ist, **dadurch gekennzeichnet, dass** als Katalysatorträger eine offenporige Schaumstruktur aus Metall, deren Stege aufgedickt wurden, oder eine Gitternetzstruktur aus Kunststoff, bestehend aus einem retikulierten, ihre Geometrie bestimmenden Schaumstoff, dessen Stege allseitig und gleichmäßig mit Kunststoffpulver beschichtet sind, verwendet wird.

2. Ozon-Katalysator nach Anspruch 1, **dadurch gekennzeichnet, dass** der metallische Katalysatorträger Beschichtungen aus Silber oder Legierungen mit Nickel-, Kupfer- und Manganbestandteilen aufweist.

3. Ozon-Katalysator nach Anspruch 1, **dadurch gekennzeichnet, dass** der Katalysatorträger aus Kunststoff eine Kunststoffpulverschicht trägt, die aus mehreren Einzelschichten aus gleichem und/oder unterschiedlichem Material besteht.

4. Ozon-Katalysator nach Anspruch 1 und 3, **dadurch gekennzeichnet, dass** das Kunststoffpulver Graphit und/oder Metall und/oder Keramik enthält.

5. Ozon-Katalysator nach Anspruch 1 und 3, **dadurch gekennzeichnet, dass** auf der Kunststoffpulverschicht eine oder mehrere Schichten aus Metall und/oder Glas aufgebracht sind.

6. Ozon-Katalysator nach Anspruch 1 und 3 bis 5, **dadurch gekennzeichnet, dass** der Schaumstoff des Katalysatorträgers aus Kunststoff ein harter Polyurethanschaumstoff ist.

7. Ozon-Katalysator nach Anspruch 1 und 2, **dadurch gekennzeichnet, dass** er in einer wasserdurchströmten Rohrleitung verwendet wird, in der er fest oder lösbar eingebracht ist und hierfür zylindrisch ausgeformt ist und einen Querschnitt, der dem der Rohrleitung entspricht, aufweist.

8. Ozon-Katalysator nach Anspruch 1 und 2, **dadurch gekennzeichnet, dass** er in der Reinigungsstufe einer technischen Wasseraufbereitung verwendet wird.

9. Ozon-Katalysator nach Anspruch 8, **dadurch gekennzeichnet, dass** er aus NiCuMn-Schaum besteht, der eine Porosität von 85 % und eine Porengröße von 4 mm aufweist, wobei er in den Kreislauf auf einer Länge von 150 mm bis 200 mm eingebaut ist.

10. Ozon-Katalysator nach Anspruch 1 und 3 bis 6, **dadurch gekennzeichnet, dass** er in einem Tischgerät zum Geruchsabbau und Entkeimen der Luft in Wohnräumen verwendet wird, wofür das versteifte Polyurethan in einem Wechselrahmen gelagert ist.

11. Ozon-Katalysator nach einen der vorstehenden Ansprüche, **dadurch gekennzeichnet, dass** er als Radiator verwendet wird, wofür versteifter Polyurethanschaum oder Aluminiumschaum mit katalytisch aktiver Schicht als Hohlzylinder ausgeformt ist.

12. Ozon-Katalysator nach Anspruch 1 und 3 bis 6, **dadurch gekennzeichnet, dass** versteifter Polyurethanschaum als Flächenelement in einem Raumteiler verwendet wird.

13. Ozon-Katalysator nach einem der Ansprüche 1 bis 6, **dadurch gekennzeichnet, dass** er in Absaugungen für Küchendünste, Rauchgase und Dämpfe, z. B. von Lösungsmitteln, verwendet wird.

14. Ozon-Katalysator nach einem der Ansprüche 1 bis 6, **dadurch gekennzeichnet, dass** als Perkolstor in einem Duschkopf oder Wasserventil verwendet wird.

15. Ozon-Katalysator nach einem der Ansprüche 1 bis 6, **dadurch gekennzeichnet, dass** er in Druckern, Scannern und Kopieren verwendet wird.

16. Ozon-Katalysator nach einem der Ansprüche 1 bis 6, **dadurch gekennzeichnet, dass** er in Schwimmbädern verwendet wird.
